(19) Europäisches Patentamt
European Patent Office
Office européen des brevets

(11) Veröffentlichungsnummer: **0 157 316 B1**

(12) **EUROPÄISCHE PATENTSCHRIFT**

(45) Veröffentlichungstag der Patentschrift: **12.08.92**

(51) Int. Cl.[5]: **C07C 45/50**, C07C 47/02

(21) Anmeldenummer: **85103428.0**

(22) Anmeldetag: **22.03.85**

Die Akte enthält technische Angaben, die nach dem Eingang der Anmeldung eingereicht wurden und die nicht in dieser Patentschrift enthalten sind.

# (54) Verfahren zur Herstellung von Aldehyden.

(30) Priorität: **03.04.84 DE 3412335**

(43) Veröffentlichungstag der Anmeldung: **09.10.85 Patentblatt 85/41**

(45) Bekanntmachung des Hinweises auf die Patenterteilung: **12.08.92 Patentblatt 92/33**

(84) Benannte Vertragsstaaten: **AT BE DE FR GB IT NL SE**

(56) Entgegenhaltungen:
**FR-A- 62 634**
**FR-A- 2 314 910**
**FR-A- 2 489 308**
**GB-A- 708 441**

(73) Patentinhaber: **HOECHST AKTIENGESELLSCHAFT**
**Postfach 80 03 20**
**W-6230 Frankfurt am Main 80(DE)**

(72) Erfinder: **Bahrmann, Helmut, Dr. Dipl.-Chem.**
**Rohstrasse 48**
**W-4236 Hamminkeln 3(DE)**
Erfinder: **Cornils, Boy, Dr. Dipl.-Chem.**
**Friedrich-Ebert-Strasse 45**
**W-4220 Dinslaken(DE)**
Erfinder: **Konkol, Werner, Dr. Dipl.-Chem.**
**Lützwostrasse 40a**
**W-4200 Oberhausen 11(DE)**
Erfinder: **Lipps, Wolfgang, Dr. Dipl.-Chem.**
**Kieningstrasse 6**
**W-7972 Isny/Allgäu(DE)**

**EP 0 157 316 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzlich und gegebenenfalls überschüssigen Liganden enthalten. Besonders bewährt haben sich als Liganden tertiäre Phosphine oder Phosphite. Ihre Anwendung ermöglicht, den Reaktionsdruck auf Werte unter 300 bar ($30 \cdot 10^3$ kPa) zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefinen mit bis zu etwa 5 Kohlenstoffatomen im Molekül beschritten werden. Außerdem hat sich gezeigt, daß die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z.B. im der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche, thermische Verfahrensschritte. Neben sulfonierten Triarylphosphinen werden als Komplexbestandteile wasserlöslicher Rhodiumkomplexverbindungen auch carboxylierte Triarylphosphine eingesetzt.

Die bekannten Verfahren haben sich ausgezeichnet bei der Hydroformylierung niederer Olefine, insbesondere Ethylen und Propylen bewährt. Setzt man höhere Olefine wie Hexen, Octen oder Decen ein, geht der Umsatz merklich zurück, so daß die Wirtschaftlichkeit der Umsetzung in technischem Maßstab nicht mehr gegeben ist.

Verursacht wird der Rückgang des Umsatzes durch die Abnahme der Löslichkeit höherer Olefine in Wasser, denn die Reaktion zwischen den Reaktanten läuft in der wäßrigen Phase ab.

Es ist zwar aus der DE 31 35 127 Al bekannt, die Hydroformylierung olefinischer Verbindungen bei Vorliegen von wäßriger und mit ihr nicht oder nur wenig mischbarer organischer Phase in Gegenwart von Lösungsvermittlern vorzunehmen.

Die praktische Durchführung dieser Umsetzung ist ausschließlich auf den Einsatz monosulfonierter bzw. monocarboxylierter Triarylphosphine als Bestandteil der Rhodiumkomplexverbindung beschränkt. Dabei zeigt sich, daß insbesondere das monosulfonierte Triphenylphosphin nur eine geringe Lebensdauer erreicht und deshalb für den wiederholten Einsatz ungeeignet ist.

Es bestand daher die Aufgabe, die vorstehend geschilderten Nachteile zu überwinden und eine Arbeitsweise zu entwickeln, die es erlaubt, auch höhere Olefine in einem aus wäßriger Katalysatorlösung und organischen Ausgangsstoffen und gegebenenfalls Reaktionsprodukten sowie gasförmigen Reaktanten bestehenden Mehrphasensystem zu hydroformylieren.

Erfindungsgemäß wird die vorstehend beschriebene Aufgabe gelöst durch ein Verfahren zur Herstellung von Aldehyden durch Umsetzung von geradkettigen oder verzweigten Olefinen mit 6 und mehr Kohlenstoffatomen mit Kohlenmonoixd und Wasserstoff in flüssiger Phase in Gegenwart von Wasser, wasserlöslichen Rhodium-Phosphin-Komplexverbindungen und Lösungsvermittlern bei Temperaturen von 20 bis 150 °C und Drücken von 1 bis 200 bar (100 bis $20 \cdot 10^3$ kPa). Es ist dadurch gekennzeichnet, daß als Katalysatoren trisulfonierte Triarylphosphine enthaltende Rhodiumkomplexverbindungen eingesetzt werden und als Lösungsvermittler Verbindungen der allgemeinen Formel

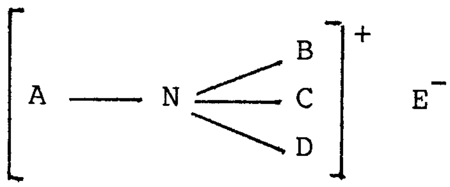

in der A für einen geradkettigen oder verzweigten Alkyl-, ω-Hydroxyalkyl-, Alkoxy- oder einen gegebenenfalls substituierten Arylrest mit jeweils 6 bis 25 Kohlenstoffatomen oder für den Rest $R^7$-CONH-$CH_2$-$CH_2$-$CH_2$, wobei $R^7$ einen geradkettigen oder verzweigten Alkylrest mit 5 bis 11 Kohlenstoffatomen bedeutet, steht, B, C und D gleich oder verschieden sind und geradkettige oder verzweigte Alkyl oder ω-Hydroxyalkylreste mit 1 bis 4 Kohlenstoffatomen bedeuten oder C und D zusammen mit N einen heterocyclischen Fünf- oder Sechsring bilden und E für Chlorid, Bromid, Jodid und insbesondere Sulfat, Tetrafluoborat, Acetat, Methosulfat, Benzolsulfonat, Alkylbenzolsulfonat, Toluolsulfonat, Lactat oder Citrat steht, verwendet werden.

Überraschenderweise gelingt die Hydroformylierung von Olefinen in Gegenwart von trisulfonierten Triarylphosphinen als Bestandteile der Rhodiumkomplexverbindung bei Anwesenheit der genannten Lösungsvermittler mit dauerhaft hohem Umsatz und hoher Selektivität, ohne daß ein Abfall infolge rascher Katalysatorzersetzung eintritt. Die als Katalysatoren eingesetzten Rhodiumverbindungen enthalten in komplexer Bindung trisulfonierte Triarylphosphine der allgemeinen Formel

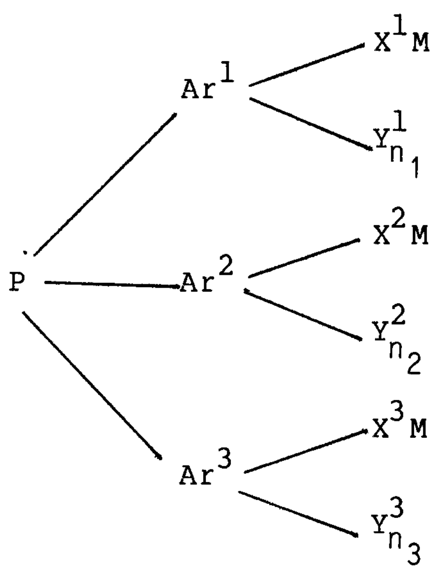

Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Sulfonat-($SO_3^-$-)Rest, $n_1$, $n_2$, $n_3$ sind gleiche oder verschiedene ganze Zahlen von 0 bis 5. M ist ein Alkalimetallion, ein Äquivalent eines Erdalkalimetall- oder Zinkions, ein Ammonium- oder quaternäres Ammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer bevorzugten Ausführungsform dieses Verfahrens enthalten die Rhodiumverbindungen als komplexbildende Phosphine Verbindungen der vorstehend beschriebenen allgemeinen Formel, in der $Ar^1$, $Ar^2$, $Ar^3$ jeweils einen Phenylrest bedeuten.

Unter dem Ausdruck Lösungsvermittler werden Stoffe oder Stoffgemische verstanden, die sowohl mit der wäßrigen, als auch mit der organischen Phase verträglich und insbesondere bei erhöhten Temperaturen in beiden Phasen löslich sind. Derartige Stoffe sind bekannt und werden auch als Phasentransfer-, obeflächenaktives- oder amphiphiles Reagenz oder als Tenside bezeichnet.

Ihre Wirkung besteht vor allem darin, daß sie die physikalischen Eigenschaften der Grenzflächen zwischen den beiden flüssigen Phasen verändern und dadurch den Übergang des organischen Reaktanten

in die wäßrige Katalysatorphase erleichtern.

Von besonderer Bedeutung ist in diesem Zusammenhang, daß der Lösungsvermittler keinen negativen Einfluß auf die Aktivität des katalytisch wirksamen Metalls hat. Nach dem chemischen Aufbau unterscheidet man anionische, kationische und nichtionogene Lösungsvermittler.

Die erfindungsgemäß eingesetzten Lösungsvermittler der allgemeinen Formel

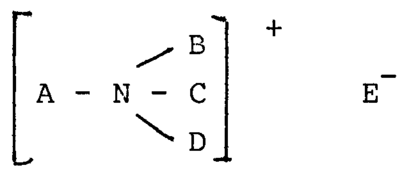

gehören zur Klasse der kationischen Phasentransferreagenzien. Bevorzugt sind als Anionen $E^-$ in der vorstehenden Formel wegen ihres geringen korrosiven Verhaltens die Methosulfate, Sulfonate und Lactate. Beispiele für geeignete Kationen

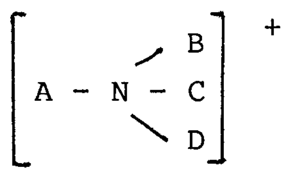

sind Stearyltrimethylammonium, Phenyltrimethylammonium, Trimethyl-1-phenyl-ammonium, Benzyltrimethylammonium, Cetyltrimethylammonium, Myristyltrimethylammonium, Dodecylpyridinium, Stearylamidomethylpyridinium, Lauryltrimethylammonium, Benzyltriethylammonium, N-(3-trimethylammoniumpropyl)n-heptansäureamidmethosulfat, Dodecyl-tris-β-hydroxyethylammonium oder N-(β-trimethylammoniumpropyl)n-nonansäureamidmethosulfat.

Die Konzentration der Lösungsvermittler in der wäßrigen Katalysatorlösung beträgt 0,5 bis 10 Gew.-%, bezogen auf die Katalysatorlösung.

Die Umsetzung des Olefins mit Wasserstoff und Kohlenmonoxid erfolgt bei Temperaturen von 20 bis 150 °C, insbesondere 50 bis 120°C und Drücken von 1 bis 200 bar (100 bis $20 \cdot 10^3$ kPa), insbesondere 10 bis 100 bar ($1 \cdot 10^3$ bis $10 \cdot 10^3$ kPa).

Der Katalysator kann dem Reaktionssystem präformiert zugesetzt werden. Mit gleich gutem Erfolg läßt er sich aber auch aus den Komponenten Rhodium oder Rhodiumverbindung und der wässrigen Lösung eines sulfonierten oder carboxylierten Triarylphosphins unter Reaktionsbedingungen im Reaktionsgemisch, also in Gegenwart des Olefins, herstellen. Außer metallischen Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wässrigen Katalysatorlösung beträgt 10 bis 2000 Gew.-ppm, bezogen auf die Lösung. Das sulfonierte bzw. carboxylierte Phosphin wird in einer solchen Menge eingesetzt, daß auf 1 Grammatom Rhodium 1 bis 1000 Mol, vorzugsweise 2 bis 300 Mol, Phosphinverbindung kommen.

Der pH-Wert der wässrigen Katalysatorlösung soll nicht unter 2 liegen. Allgemein stellt man einen pH-Wert von 2 bis 13, vorzugsweise 4 bis 10, ein.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das erfindungsgemäße Verfahren wird mit Erfolg bei der Hydroformylierung von geradkettigen oder verzweigten Olefinen mit vier und mehr, insbesondere mit sechs und mehr Kohlenstoffatomen angewandt. Die Doppelbindung in diesen Olefinen kann end- oder innenständig sein.

Die folgenden Beispiele erläutern die Erfindung näher ohne sie auf die beschriebenen Ausführungsformen zu beschränken. Zur Charakterisierung der Leistungsfähigkeit der Katalysatorsysteme wird neben dem Verhältnis von n-Aldehyd zu i-Aldehyd der Begriff der "Aktivität" definiert als

$$\frac{\text{mol Aldehyde}}{\text{g-Atom Rh} \cdot \text{min}}$$

und "Produktivität" definiert als

$$\frac{\text{g-Aldehyde}}{\text{cm}^3\ \text{Katalysatorlösung} \cdot \text{h}}$$

verwendet. Die Alkohol- und Kohlenwasserstoffbildung ist minimal.

Beispiel 1 ist ein Vergleichsversuch, der ohne Zusatz eines Lösungsvermittlers durchgeführt wurde.

Beispiel 1 (Vergleichsbeispiel)

a) Katalysatorpräformierung

In einem 1 l Autoklaven mit Tauchstutzen werden 345 ml einer wässrigen Lösung von Trinatrium-tri(m-sulfophenyl)-phosphin mit einem Gehalt von 20,4 Gew.% Salz sowie 400 ppm Rh als Rh-Acetat vorgelegt; Synthesegas ($CO/H_2$-Vol.-verhältnis 1 : 1) wird bis zu einem Druck von 25 bar ($2,5 \cdot 10^3$ kPa) aufgepreßt. Danach wird die Reaktionslösung 3 h unter Rühren bei 125°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung (~10 g) über den Tauchstutzen herausgedrückt und analysiert. Die restliche Lösung verbleibt im Autoklaven.

b) Hydroformylierung

In die nach a) hergestellte Lösung werden unter Rühren 170 g n-Hexen-l gepumpt. Bei konstantem Druck von 25 bar ($2,5 \cdot 10^3$ kPa) wird das Gemisch auf 125°C erhitzt und 3 h bei dieser Temperatur belassen. Danach kühlt man auf 30°C ab und läßt absitzen. Die überstehende organische Phase wird über den Tauchstutzen herausgedrückt; sie wird gewogen (s. Tabelle 1) und gaschromatographisch untersucht.

Der Teilschritt b) wird insgesamt dreimal wiederholt, wobei im wesentlichen die gleichen Ergebnisse resultieren. Die in der Tabelle 1 aufgeführten Werte für Aktivität und Produktivität beziehen sich auf die im Autoklaven vorhandenen Mengen wäßriger und organischer Phase. Das spezifische Gewicht der wäßrigen Phase beträgt 1,1304.

Tabelle 1

| Anzahl der Hydroformylierungen | 1 | 2 | 3 | 4 | ∅ |
|---|---|---|---|---|---|
| Umsatz (% nach GC) | 22 | 18 | 18 | 16 | 18 |
| n/i-Verhältnis | 98/2 | 98/2 | 98/2 | 98/2 | 98/2 |
| organische Phase (g) | 153 | 167 | 172 | 175 | 167 |
| wäßrige Phase im Reaktor (g) | 346 | 344 | 343 | 342 | 344 |
| Aktivität $\left[\frac{\text{mol } C_7\text{-aldehyde}}{\text{g-Atom Rh} \cdot \text{min}}\right]$ | 1,22 | 1,09 | 1,13 | 1,02 | 1,11 |
| Produktivität $\left[\frac{\text{g-}C_7\text{-Aldehyde}}{\text{cm}^3 \text{ Katalys.lösg.} \cdot \text{h}}\right]$ | 0.037 | 0.033 | 0.034 | 0.031 | 0.033 |

Beispiel 2

Beispiel 1 wird wiederholt mit dem Unterschied, daß entsprechend der Erfindung der wäßrigen Katalysatorlösung zusätzlich 9,75 g Tetradecyl-trimethylammoniummethosulfat (2,5 Gew.%) zugegeben wird. Das spezifische Gewicht der Katalysatorlösung beträgt 1,171.

Die Versuchsergebnisse sind in Tabelle 2 zusammengestellt.

Tabelle 2

| | 1 | 2 | 3 | 4 | 5 | Ø |
|---|---|---|---|---|---|---|
| Umsatz (% nach GC) | 41 | 47 | 48 | 35 | 42 | 43 |
| n/i-Verhältnis | 95/5 | 96/4 | 96/4 | 96/4 | 96/4 | 96/4 |
| organische Phase (g) | 168 | 175 | 185 | 220 | 176 | 185 |
| wäßrige Phase im Reaktor (g) | 374 | 352 | 341 | 336 | 306 | 342 |
| Aktivität $\left[\frac{\text{mol } C_7\text{-aldehyde}}{\text{g-Atom Rh} \cdot \text{min}}\right]$ | 2.31 | 2.93 | 3.23 | 2.87 | 3.02 | 2.87 |
| Produktivität $\left[\frac{\text{g-}C_7\text{-aldehyde}}{\text{cm}^3 \text{ Kat.lösg.} \cdot \text{h}}\right]$ | 0.072 | 0.091 | 0.102 | 0.089 | 0.094 | 0.090 |

Beispiel 2 zeigt, daß Aktivität und Produktivität durch Zusatz eines Lösungsvermittlers deutlich verbessert werden, ohne daß die Selektivität wesentlich schlechter wird.

Zur Bilanzierung des Austrags an Phosphor und Rhodium werden die organischen Produkte des Beispiels 2 vereinigt und nach Einengung analysiert. Im organischen Produkt sind 0,3 ppm Rhodium und 4 ppm Phosphor enthalten.

In den folgenden Beispielen 3 bis 9 wird die Wirkung verschiedener Lösungsvermittler veranschaulicht. Die Ergebnisse von fünf Hydroformylierungen mit der gleichen Katalysatorlösung sind jeweils als Durchschnittswert in Tabelle 3 zusammengefaßt. Die Versuchsbedingungen sind die gleichen wie in den Beispielen 1 und 2.

Tabelle 3

| Beispiel-Nr. bzw. Zusatz (s.u.) | 1 | 3 | 4 | 5 | 6 | 7 | 8 | 9 |
|---|---|---|---|---|---|---|---|---|
| Menge (%) | - | 1 | 1 | 1 | 2,5 | 2,5 | 2,5 | 2,5 |
| Umsatz (% nach GC) | 18 | 23 | 25 | 27 | 30 | 30 | 35 | 33 |
| Aktivität $\left[\frac{\text{mol } C_7\text{-aldehyde}}{\text{g-Atom Rh} \cdot \text{min}}\right]$ | 1,07 | 1,43 | 1,58 | 1,74 | 1,88 | 2,28 | 2,41 | 2,36 |
| Produktivität $\left[\frac{\text{g-}C_7\text{-aldehyde}}{\text{cm}^3 \text{ Katalysatorlösg.} \cdot \text{h}}\right]$ | 0,032 | 0,045 | 0,049 | 0,054 | 0,056 | 0,069 | 0,072 | 0,071 |

3. Olylalkohol (HD-Ocenol 150/170 Hersteller: Henkel KG)
4. Lauryltrimethylammoniumchlorid
5. Laurylpyridiniumchlorid
6. Benzyltrimethylammoniumsulfat
7. Dodecyl-trimethylammoniummethosulfonat
8. Benzyltrimethylammoniumlactat
9. Dimethylethylhexadecylammoniumbenzolsulfonat

In den folgenden Beispielen wird die Konzentration des wasserlöslichen, trisulfonierten Phosphins vermindert, so daß der Gehalt an Tri(m-sulfophenyl)phosphin-Na-Salz 12,2 Gew.% beträgt. Ansonsten wird wie in den Beispielen 1 bis 2 verfahren. Es werden wiederum die Durchschnittswerte von fünf Hydroformylierungen mit der gleichen Katalysatorlösung zusammengefaßt. Die Ergebnisse sind in Tabelle 4 zusammengestellt.

Tabelle 4

| Beispiel/ Zusatz-Nr. | 10 ohne | 11 | 12[a)] | 13 | 14 | 15 | 16 | 17 |
|---|---|---|---|---|---|---|---|---|
| Konzentr. (%) | - | 5 | 2.5 | 0.5 | 2.5 | 2.5 | 2.5 | 2.5 |
| Umsatz (% nach GC) | 36 | 43 | 86 | 43 | 43.5 | 39 | 42 | 50 |
| Aktivität $\left[\frac{\text{mol } C_7\text{-aldehyde}}{\text{g-Atom Rh} \cdot \text{min}}\right]$ | 1.94 | 3.40 | 6.1 | 2.7 | 2.8 | 2.47 | 3.3 | 3.8 |
| Produktivität $\left[\frac{\text{g-}C_7\text{-aldehyde}}{\text{cm}^3 \text{ Katalysatorlösg.} \cdot \text{h}}\right]$ | 0.065 | 0.098 | 0.200 | 0.087 | 0.088 | 0.072 | 0.097 | 0.112 |

a) ∅ 30 Einzelversuche

11. Polyglycol 200 (Hersteller: Hoechst AG)
12. Trimethyl-hexadecylammoniumbromid
13. Trimethyl-hexadecylammoniumbromid
14. Triethylenglycol
15. Na-Salze der Ölsäure
16. Sulfolan
17. Tributylhexadecylammoniumlactat

Der Vergleich der Tabellen 3 und 4 zeigt den Einfluß des Phosphor-Rhodium-Verhältnisses auf die Höhe des Umsatzes bei der Hydroformylierung. Es wird deutlich, daß bereits das Grundniveau der Aktivität mit etwa 2 bei niedrigerem Rh/P-Verhältnis von etwa 1 : 50 (Tabelle 4) etwa doppelt so hoch liegt wie bei 1 : 100 (Tabelle 3) mit 1. Die bei niedrigem P/Rh-Verhältnis erzielbare Umsatzsteigerung durch den erfindungsgemäßen Zusatz der Lösungsvermittler geht noch darüber hinaus.

Zur Bilanzierung des Austrags an Phosphor und Rhodium werden die organischen Produkte des Beispiels 12 vereinigt und nach Einengen analysiert. Im organischen Produkt sind 0.41 ppm Rhodium und 6.63 ppm Phosphor enthalten.

## Patentansprüche

1. Verfahren zur Herstellung von Aldehyden durch Umsetzung von geradkettigen oder verzweigten Olefinen mit 6 und mehr Kohlenstoffatomen mit Kohlenmonoixd und Wasserstoff in flüssiger Phase in Gegenwart von Wasser, wasserlöslichen Rhodium-Phosphin-Komplexverbindungen und Lösungsvermittlern bei Temperaturen von 20 bis 150 °C und Drücken von 1 bis 200 bar (100 bis 20 • $10^3$ kPa), dadurch gekennzeichnet, daß als Katalysatoren trisulfonierte Triarylphosphine enthaltende Rhodiumkomplexverbindungen eingesetzt werden und als Lösungsvermittler Verbindungen der allgemeinen Formel

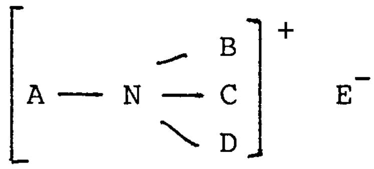

in der A für einen geradkettigen oder verzweigten Alkyl-, ω-Hydroxyalkyl-, Alkoxy- oder einen gegebenenfalls substituierten Arylrest mit jeweils 6 bis 25 Kohlenstoffatomen oder für den Rest $R^7$-CONH-$CH_2$-$CH_2$-$CH_2$, wobei $R^7$ einen geradkettigen oder verzweigten Alkylrest mit 5 bis 11 Kohlenstoffatomen bedeutet, steht, B, C und D gleich oder verschieden sind und geradkettige oder verzweigte Alkyl oder ω-Hydroxyalkylreste mit 1 bis 4 Kohlenstoffatomen bedeuten oder C und D zusammen mit N einen heterocyclischen Fünf- oder Sechsring bilden und E fur Chlorid, Bromid, Jodid und insbesondere Sulfat, Tetrafluoborat, Acetat, Methosulfat, Benzolsulfonat, Alkylbenzolsulfonat, Toluolsulfonat, Lactat oder Citrat steht, verwendet werden.

2. Verfahren nach Patentanspruch 1, dadurch gekennzeichnet, daß die als Katalysatoren eingesetzten Rhodiumverbindungen in komplexer Bindung trisulfonierte bzw. tricarboxylierte Triarylphosphine der allgemeinen Formel

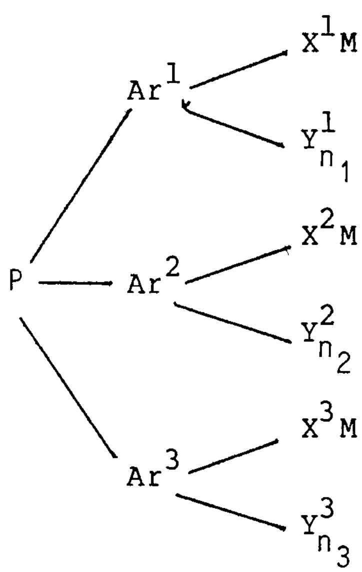

enthalten, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen bedeuten, $X^1$, $X^2$, $X^3$ jeweils ein Sulfonat-($SO_3^-$-

)Rest ist, $n_1$, $n_2$, $n_3$ gleiche oder verschiedene ganze Zahlen von 0 bis 5 sind und M ein Alkali-, Erdalkalimetall-, Zink-, Ammonium- oder quaternäres Ammoniumion der allgemeinen Formel N-$(R^3R^4R^5R^6)^+$ in der $R^3$, $R^4$, $R^5$, $R^6$ jeweils für eine geradkettige oder verzweigte Alkylgruppe mit 1 bis 4 C-Atomen steht, ist.

3. Verfahren nach Anspruch 2, dadurch gekennzeichnet, daß $Ar^1$, $Ar^2$, $Ar^3$ jeweils einen Phenylrest bedeuten.

4. Verfahren nach Anspruch 1 bis 3, dadurch gekennzeichnet, daß die Konzentration des Lösungsvermittlers in der wäßrigen Katalysatorlösung 0,5 bis 10 Gew.-%, bezogen auf die Katalysatorlösung, beträgt.

**Claims**

1. A process for the preparation of aldehydes by reacting straight-chain or branched olefins having 6 or more carbon atoms with carbon monoxide and hydrogen in the liquid phase in the presence of water, water-soluble rhodium-phosphine complex compounds and solubilisers at temperatures of 20 to 150 ° C and at pressures of 1 to 200 bar (100 to 20 x $10^3$ kPa), characterised in that the catalysts used are rhodium complex compounds containing trisulfonated triarylphosphines and the solubilisers used are compounds of the general formula

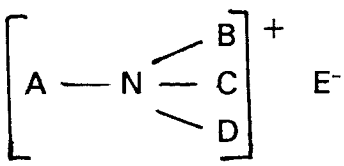

where A stands for a straight-chain or branched alkyl, ω-hydroxyalkylor alkoxy radical or an optionally substituted aryl radical each having 6 to 25 carbon atoms or for the radical $R^7$-CONH-$CH_2$-$CH_2$-$CH_2$, where $R^7$ is a straight-chain or branched alkyl radical having 5 to 11 carbon atoms, B, C, D are the same or different and stand for straight-chain or branched alkyl or ω-hydroxyalkyl radicals having 1 to 4 carbon atoms or C and D together with N form a heterocyclic five or six-membered ring and E stands for chloride, bromide, iodide and in particular sulfate, tetrafluoroborate, acetate, methosulfate, benzene sulfonate, alkylbenzene sulfonate, toluene sulfonate, lactate or citrate.

2. A process according to claim 1, characterised in that the rhodium complex compounds used as catalysts contain trisulfonated and/or tricarboxylated triarylphosphines of the general formula

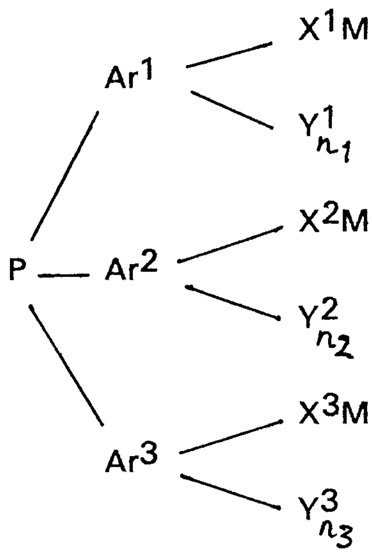

where $Ar^1$, $Ar^2$, $Ar^3$ each stand for a phenyl or naphthyl group, $Y^1$, $Y^2$, $Y^3$ each denote a straight-chain or

branched alkyl group having 1 to 4 carbon atoms, an alkoxy group, a halogen atom, the OH, CN, $NO_2$ or $R^1R^2N$ groups, where $R^1$ and $R^2$ each stand for a straight-chain or branched alkyl group having 1 to 4 carbon atoms, $X^1$, $X^2$, $X^3$ are each a sulfonate-($SO_3^-$) radical, $n_1$, $n_2$, $n_3$ are the same or different integers from 0 to 5 and M is an alkali metal ion, alkaline earth metal ion, zinc ion, ammonium ion or quaternary ammonium ion of the general formula $N(R^3R^4R^5R^6)^+$ where $R^3$, $R^4$, $R^5$, $R^6$ each stand for a straight-chain or branched alkyl group having 1 to 4 carbon atoms.

**3.** A process according to claim 2, characterised in that $Ar^1$, $Ar^2$ and $Ar^3$ each denote a phenyl radical.

**4.** A process according to claims 1 to 3, characterised in that the concentration of the solubiliser in the aqueous catalyst solution is 0.5 to 10 wt %, related to the catalyst solution.

**Revendications**

**1.** Procédé pour la fabrication d'aldéhydes par réaction d'oléfines à chaîne droite ou ramifiée en $C_6$ et plus avec le monoxyde de carbone et l'hydrogène en phase liquide en présence d'eau, de composés complexes rhodiumphosphine solubles dans l'eau et d'agents solubilisants à des températures de 20 à 150 °C et sous des pressions de 1 à 200 bar (100 à $20.10^3$ kPa), caractérisé en ce que l'on utilise comme catalyseurs des composés complexes de rhodium contenant des triarylphosphines trisulfonées et comme agents solubilisants des composés de formule générale

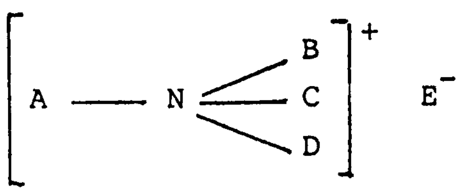

dans laquelle A représente un reste alkyle, ω-hydroxyalkyle, alcoxy à chaîne droite ou ramifiée ou un reste aryle éventuellement substitué chaque fois en $C_6$-$C_{25}$ ou le reste $R^7$-CONH-$CH_2$-$CH_2$-$CH_2$, dans lequel $R^7$ représente un reste alkyle à chaîne droite ou ramifiée en $C_5$-$C_{11}$, B, C et D sont identiques ou différents et représentent des restes alkyles ou ω-hydroxyalkyles à chaîne droite ou ramifiée en $C_1$-$C_4$, ou bien C et D forment avec N un noyau hétérocyclique à 5 ou 6 chaînons et $E^-$ représente un anion chlorure, bromure, iodure et en particulier sulfate, tétrafluoroborate, acétate, méthylsulfate, benzènesulfonate, alkylbenzènesulfonate, toluènesulfonate, lactate ou citrate.

**2.** Procédé selon la revendication 1, caractérisé en ce que les composés de rhodium utilisés comme catalyseurs contiennent en liaison complexe des triarylphosphines trisulfonées de formule générale

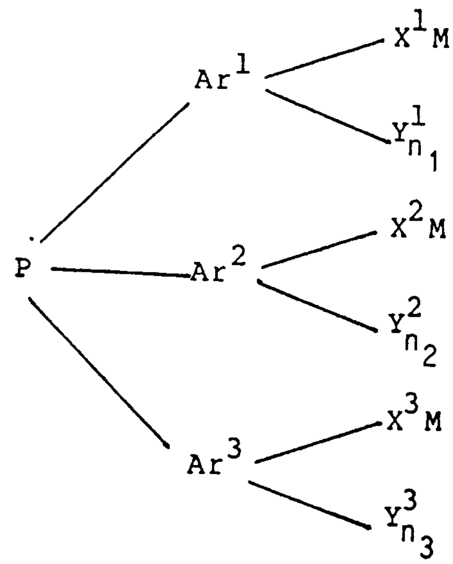

dans laquelle $Ar^1$, $Ar^2$ et $Ar^3$ représentent chacun un groupe phényle ou un groupe naphtyle, $Y^1$, $Y^2$ et $Y^3$ représentent chacun un groupe alkyle à chaîne droite ou ramifiée en $C_1$-$C_4$, un groupe alcoxy, un atome d'halogène, le groupe OH, le groupe CN, le groupe $NO_2$ ou le groupe $R^1R^2N$ dans lequel $R^1$ et $R^2$ représentent chacun un groupe alkyle à chaîne droite ou ramifiée en $C_1$-$C_4$ ; $X^1$, $X^2$ et $X^3$ représentent chacun un reste sulfonate ($SO_3^-$), $n_1$ et $n_2$ et $n_3$ sont des nombres entiers identiques ou différents de 0 à 5. M est un ion de métal alcalin, un équivalent d'ion de métal alcalino-terreux ou d'ion zinc, un ion ammonium ou un ion ammonium quatermaire de formule générale $N(R^3R^4R^5R^6)^+$ dans laquelle $R^3$, $R^4$, $R^5$ et $R^6$ représentent chacun un groupe alkyle à chaîne droite ou ramifiée en $C_1$-$C_4$.

**3.** Procédé selon la revendication 2, caractérisé en ce que $Ar^1$, $Ar^2$ et $Ar^3$ représentent chacun un reste phényle.

**4.** Procédé selon les revendications 1 à 3, caractérisé en ce que la concentration de l'agent solubilisant dans la solution aqueuse de catalyseur est de 0,5 à 10 % en poids par rapport à la solution de catalyseur.